# EUROPEAN PATENT APPLICATION

(11) **EP 2 246 440 A1**
(43) Date of publication of application: **03.11.2010**
(21) Application number: 09305379.1
(22) Date of filing: 29.04.2009
(51) Int. Cl.: C12Q 1/46, G01N 33/543, G01N 33/573

(54) **Enzymatic assay for the quantitative determination of phospholipase a1 or a2 activity in a sample**

(71) Applicant: Aterovax, 75014 Paris (FR)
(72) Inventor: Valentin, Emmanuel, 75014 Paris (FR); Sibella, Carla, 75014 Paris (FR)
(74) Representative: Icosa

(57) **Abstract**

The invention relates to a new method for measuring sPLA2 activity and kit for use thereof.

## Description

### FIELD OF THE INVENTION

The invention relates to an enzymatic assay for the quantitative determination of phospholipase activity in a sample.

### BACKGROUND OF THE INVENTION

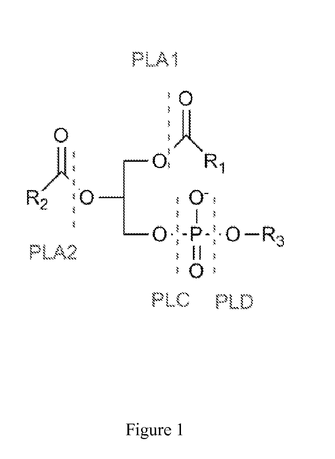

Phospholipids are the primary structural constituents of biological membranes. In addition to this structural role, the importance of phospholipids as mediators in cellular signaling processes has become increasingly apparent. Consequently, research into metabolic processes such as phospholipase action and lipid sorting and trafficking is rapidly expanding.
Phospholipases belong to one of the most familiar interfacial enzymes family that express their catalytic activity once present in an interfacial system. Phospholipases also belong to the class of hydrolytic enzymes, however they cleave favorably and specifically the ester linkage of phospholipids present in aqueous systems, to yield free fatty acids, lysophosholipids, glycerophospholipids, phosphatidic acid and free alcohol, depending on the type of phospholipase. These interfacial enzymes are not classical soluble enzymes that access their substrate directly from the aqueous phase as their enzymatic kinetic path is composed of two distinct steps: 1) binding to the organized substrate 2) catalysis step. Phospholipases are classified according to their site of action in the phospholipid molecule (Figure 1) (11).

Thus, a phospholipase A1 (PLA1) hydrolyzes the 1-acyl group of a phospholipid, i.e. it hydrolyzes the bond between the fatty acid and the glycerine residue at the 1-position of the phospholipid. A phospholipase A2 (PLA2) hydrolyzes the 2-acyl, or central acyl, group and phospholipases C (PLC) and D (PLD), which are also known as phosphodiesterases, cleave on different sides of the phosphodiester linkage.
The hydrolysis of a phospholipid by a PLA1 or a PLA2 results in the production of a so-called "lysophospholipid". Selective hydrolysis of a phospholipid substrate with a PLA1 produces a 2-acyl lysophospholipid and selective hydrolysis of a phospholipid with a PLA2 results in the production of a 1-acyl lysophospholipid. The phospholipase metabolites are involved in diverse cellular processes including signal transduction, host defense (including antibacterial effects), formation of platelet activating cofactor, membrane remodeling and general lipid metabolism.
Secreted phospholipase A2 (sPLA2) are disulfide rich, 14-kDa, Ca2+-dependent, extracellular enzymes. They are distinct from the lipoprotein-associated phospholipase A2 (Lp-PLA2), also know as PAF-acetyl hydrolase. Nine different sPLA2 genes have been described in human and classified into group IB, IIA, IID, IIE, IIF, III, V, X and XII. The group IB and IIA enzymes are best characterized but the biological roles played by the more recently discovered members of the family remain elusive.
Recently, nine large independent international clinical studies, both in clinical and non clinical population demonstrated the strong positive correlation existing between sPLA2 and atherosclerotic cardiovascular disease (1-9). More specifically, sPLA2 activity emerged as a new powerful tool for: risk stratification in acute coronary syndrome management, acute coronary syndrome diagnosis, cardiovascular risk assessment in non symptomatic patients and theragnostic and monitoring of therapeutics in cardiovascular diseases.
The PLA2 group also comprises cytosolic PLA2, whose activity on PC liberates arachidonic acid, a precursor for the biosynthesis of prostaglandins and leukotrienes and possible intracellular secondary messenger.
Phospholipase A₁ (PLA₁) is an enzyme that hydrolyzes phospholipids and produces 2-acyllysophospholipids and fatty acids and is conserved in a wide range of organisms. Mammals have several enzymes that exhibit PLA₁ activity *in vitro.* The extracellular PLA₁s include phosphatidylserine (PS)-specific PLA₁ (PS-PLA₁), membrane-associated phosphatidic acid (PA)-selective PLA_{1S} (mPA-PLA₁α and mPA-PLA₁β), hepatic lipase (HL), endothelial lipase (EL) and pancreatic lipase-related protein 2 (PLRP2), all of which belong to the pancreatic lipase gene family. The former three PLA₁s differ from other members in their substrate specificities, structural features and gene organizations, and form a subfamily in the pancreatic lipase gene family. PS-PLA₁, mPA-PLA₁α and mPA-PLA₁β exhibit only PLA₁ activity, while HL, EL and PLRP2 show triacylglycerol-hydrolyzing activity in addition to PLA₁ activity. An assay for serum PLA2 (13) utilized liquid-liquid phase partition in the analysis of fluorogenic reaction products. These assays are laborious, requiring time-consuming post-reaction analyses.
US2003/219849 disclosed a general method for measuring the activity of a phospholipase, based on the use of a liposome that comprises a nonfluorescent phosphatidylcholine, a nonfluorescent negatively charged phospholipid selected from phosphatidylglycerol, phosphatidylserine, phosphatidylinositol or phosphatidic acid and a fluorescent labelled molecule selected among a fluorescently labelled phosphatidylcholine and a fluorescently labelled/nagetively charged phospholipid. The hydrolysis of the phospholipids components of the liposome by a phospholipase causes a fluorescence intensity change. One example for determining a sPLA2 activity is based on the use of a liposome that contains dioleoyl phosphatidylcholine, phosphatidylglycerol and fluorescently labelled 1,2-bis-(4,4-difluoro-5,7-dimethyl-4-bora-3a,4a-diaza-s-indecene-3-undecanoyl)-sn-glycero-3-phosphocholine (Bis-BODIPY FL C11-PC).
US2005/026235 disclosed another general method for determining phospholipase activity, said method being based on the use of a lipid complex comprising a fluorescently-labelled phospholipase substrate in the form of a micelle or a liposome, said substrate having the formula: 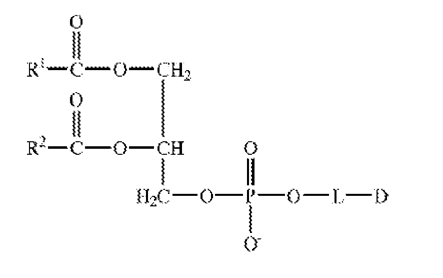 wherein R1 is a saturated or unsaturated alkyl having from 6 to 30 carbon atoms,
R2 is a saturated or unsaturated alkyl having from 6 to 30 carbon atoms,
L is a bond or a linker, and D is a fluorescent fluorophore.
However, the inventors found that having the phospholipase's substrate in the form of liposome or micelle presents several drawbacks. Indeed, because naturally occurring glycerolipid or glycerophospholipid have virtually no solubility in water and because interfacial enzyme can exist in water-soluble state, the enzyme must adsorb to the substrate membrane interface for glycerolipid or glycerophospholipid hydrolysis to occur. Although seemingly simple, the hydrolysis of a glycerolipid or glycerophospholipid bilayer is a highly complex, nonequilibrium process. The rapid production of fatty acid or fatty acid and lysophospholipid by phopholipases creates a complex membrane dynamic that in turn, will be self perturbing and affect continued enzyme actions. Indeed, phospholipase activity is sensitive to the membrane character and is modified by a host of membrane factors that include the phospholipid headgroup size, headgroup charge, phospholipid phase state, phospholipid dynamics and membrane curvature.

Another methods for detecting sPLA2 activity have been reported (Kit, Cat#765001 sPLA2 Assay Kit product brochure, Cayman Chemical, Ann Arbor, MI, 12/18/97 and kit #907-002, sPLA2 Assay kit, Assay design, Inc.5777 Hines Drive, Ann Arbor, Michigan 48108 USA).
These assay formats have major limitations. For example, assays which measure only enzymatic activity suffer from competitive activity for the substrate by other enzymes or substances present in the test sample. For instance, many members of the phospholipase A2 family show enzymatic activity toward oxidized phosphatidylcholine.
Additionally, the Cayman and Assay design activity assays suffer from background signal due to substances in serum which convert the substrate independent of sPLA2 activity. Specifically, these kits relie on the detection of free thiol as part of the methodology. While the Cayman assay may work well in a laboratory setting, detecting free thiols makes the Cayman kit not suitable for use to measure sPLA2 in human samples because of the abundant free thiols in human tissue, plasma or serum samples. In addition, existing assays may detect erroneously high activity due to the lack of specificity. False measurements of activity in a clinical setting may lead to improper diagnosis of disease, or a patient's response to a therapy intended to reduce enzymatic activity.

Thus, using a substrate in the form of liposome/micelle presents drawbacks as liposome/micelle are not stable structures in solution and exist in multiple forms: small to large, multilamellar (liposomes inside other liposomes) to unilamellar vesicules.
In addition, the production of liposomes by dropping lipid solution in organic solvent into aqueous solutions is a non-reproducible process, leading to great variations in the nature and organization of the produced liposome. Thus, the quantity of substrate accessible for the enzyme, which is the external leaflet of the liposome, will be variable if the substrate is prepared that way. The difficulty of controlling liposome size makes also standardization of the liposome preparation a problem.
Moreover, several factors present in human serum sample can greatly influence the structure of the liposomes used as substrate in phospholipase enzymatic assays and therefore influence the measurement of phospholipase activity. Said factors are for example the ionic strength, the presence of lipids or molecules acting as detergents, or the divalent ions concentrations in the sample.
Therefore, when dealing with very complex samples, such as biological samples, it is crucial to:
1) minimize the influence of the phospholipase activity independent factors to the substrate, and
2) be able to measure as accurately as possible the phospholipase activity independent factors, and to substrate them from the interfacial enzyme activity itself.

Cho et al. (12) disclosed an assay for determining sPLA2 activity based on the use of radiolabaled phospholipids coated beads. Said beads were cotaed with [3H]-POPG (1-pamiltoyl-2-oleoyl-sn-glycero-3-phosphoglycerol) at an equilibrium packing density of 1.3 10⁻² molecules / A², i.e. 1.3 molecules / nm². This assay presents the following drawbacks:
(i) radiolabelling is not suitable for diagnostic application, (ii) as shown in the examples of the present invention, the packing density of 1.3 molecules / nm² is not suitable for use in a fluorometric assay, and (iii) the detection limit of said method is of 1 ng.

Therefore, a more accurate phospholipase A1 or A2 activity assay is of great interest, especially for testing complex samples in the field of diagnostic.

Thus, there is a need for a new phospholipase A1 or A2 activity assay that is more sensitive, reproducible, and specific.

### SUMMARY OF THE INVENTION

One object of the invention is a method for measuring a calcium-dependent phospholipase A1 or A2 enzymatic activity in a sample, comprising the following steps:
a) contacting said sample with a solid phase coated with a fluorochrome-labelled phospholipase A1 or A2 substrate, wherein the molecular coverage is in the range 8 to 11 molecules / nm2 in conditions where calcium-dependent phospholipase A1 or A2 enzymatic activity is blocked.
b) reading the fluorescence emission overtime
c) adding a solution to initiate calcium-dependent phospholipase A1 or A2 enzymatic activity
d) reading the fluorescence emission overtime.
In one embodiment of the invention, the sample is selected from urine, serum, plasma sample, whole blood, bronchoalveolar lavage fluid, sputum, synovial fluid, amniotic fluid, peritoneal fluid, cerebrospinal fluid, pleural fluid, pericardial fluid and alveolar macrophages.
In another embodiment of the invention, step a) is performed in the presence of at least one divalent ions chelating agent.
In another embodiment of the invention, the concentration of the divalent ions chelating agent is from 1 mM to 20 mM.
In another embodiment of the invention, the solution that initiates calcium-dependent phospholipase A1 or A2 enzymatic activity comprises an excess of Ca2+ divalent ions.
In another embodiment of the invention, the concentration of Ca2+ divalent ions in the solution is from 1 to 10 mM.
In another embodiment of the invention, said substrate comprises a hydrophobic moiety, a phosphate moiety and a fluorescent moiety attached to the hydrophobic moiety either directly or via an optional linker.
In another embodiment of the invention, said substrate is a glycerophospholipid.
In another embodiment of the invention, said substrate is labelled on the terminal end of the sn-1 fatty acyl chain and/or on the terminal end of the sn-2 fatty acyl chain.
In another embodiment of the invention, the labelled substrate is labelled on the sn-1 fatty acid chain with a donor fluorophore and on the sn-2 fatty acid chain with an acceptor fluorophore or inversely.
In another embodiment of the invention, the labelled substrate is labelled on the sn-1 fatty acid chain with a fluorophore and on the sn-2 fatty acid chain with an quencher or inversely.
Another object of the invention is a kit comprising:
- a first container comprising a solid phase coated with a fluorochrome-labelled phospholipase A1 or A2 substrate, wherein the molecular coverage is in the range 8 to 11 molecules / nm2,
- a second container comprising an assay buffer comprising at least a buffer solution. one or more divalent ions chelating agent, and bovine serum albumin.
- a third container comprising a solution that initiates calcium-dependent phospholipase A1 or A2 enzymatic activity, said solution comprising Ca2+ divalent ions.
In one embodiment of the invention, the kit further comprises controls and/or calibrators.
Another object of the invention is a method of identifying a subject having or at risk of having or developing a sPLA2 activity-linked disease, comprising measuring the sPLA2 enzymatic activity in a sample from the subject according to the method as described here above.
In one embodiment of the invention, said sPLA2 activity-linked disease is a cardiovascular disease and/or a cardiovascular event.

### DETAILED DESCRIPTION OF THE INVENTION

The phospholipase activity assay of the invention is a continuous, fluorimetric, kinetic activity assay. It is based on the measurement over time of the hydrolysis of a fluorochome-labelled substrate molecule by phospholipases present in a sample, said fluorochrome-labelled substrate being coated on a solid phase, for example microspheres or beads.

One object of the invention is a solid phase coated with a fluorochrome-labelled substrate, wherein the molecular coverage is in the range from 8 to 11 molecules per square nanometer, preferably from 8 to 11 molecules per square nanometer, more preferably from 8 to 11 molecules per square nanometer. A method to determine molecular coverage of the beads is shown in the examples.

As used herein, the term "molecular coverage" refers to the number of molecule per square nanometer.
As used herein, the term "solid phase coated with" refers to any support to which the substrate can be bound (e.g. coated, attached, immobilized, entrapped or captured) or which can be functionalized to accept the substrate. The substrate is coated such that it is accessible to the phospholipases in solution.
The solid phase can be composed of a natural or synthetic material, an organic or inorganic material, such as polymer, resin, metal or glass, and combinations thereof.
According to the invention, said labelled substrate is stabilised on a solid phase. In one embodiment, said labelled substrate is attached non-covalently to the solid phase.
Examples of solid phase include, but are not limited to, microparticles such as latex (polystyrene) beads, glass beads, magnetic beads, iron beads, gold beads; or a sheet, a matrix or a sample receptacle. Examples of sample receptacles include sample wells, tubes, plates or vials.
In one embodiment of the invention, said labelled substrate is stabilised on beads, preferably latex beads.
Said beads are of conventional size: for example 0.5 to 5 µm, preferably 3-5 µm, more preferably 4-4.5 µm.

According to the invention, the fluorescently-labelled phospholipase substrate is cleavable by a phospholipase selected from a phospholipase A1 and a phospholipase A2.
In one embodiment of the invention, said substrate comprises a hydrophobic moiety, a phosphate moiety and a fluorescent moiety attached to the hydrophobic moiety either directly or via an optional linker.
In one embodiment of the invention, said substrate is a glycerophospholipid that may be anionic, cationic or neutral. Preferably, said glycerophospholipid is anionic.

Said glycerophospholipid has the following formula: 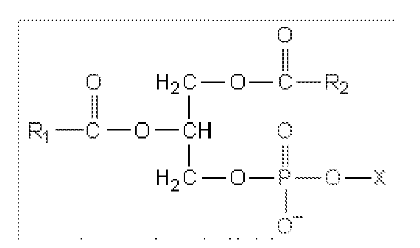 The hydrophobic moiety of the substrate can include one or more hydrophobic tails, such as R1 and R2, wherein R1 and R2 can be the same or different. The exact length and/or composition of each hydrophobic tail can be selectively varied. In some embodiments, R1 and R2 may be each the same or different saturated or unsaturated C6, C7, C8, C9, C10, C11, C12, C13, C14, C15, C16, C17, C18, C19, C20, C22, C24 or C26 n-alkyl chain. Each hydrophobic tail may comprise alkyl, aryl, or arylalkyl groups, or combinations of such groups. The alkyl groups, or the alkyl portion of the arylalkyl groups, may be saturated or unsaturated, and may be linear, branched, cyclic or combinations of linear, branched and cyclic, in conformation. The aryl groups or the aryl portions of the arylalkyl groups may be monocyclic, polycyclic or bi-, or tri-, cyclic, etc. The hydrophobic moiety may comprise two, three or even more hydrophobic tails. The carbon atoms comprising the hydrophobic moiety may be substituted with various substituents. The chains comprising the hydrophobic moiety may be attached to the remainder of the molecule via virtually any type of linkage, provided that the resultant labeled phospholipase cleavage substrate is cleavable by the particular phospholipase being assessed.
In one embodiment, when phospholipase A1 activity is being assessed, the hydrophobic tail R1 must be connected to the remainder of the molecule by an ester linkage.
In another embodiment, when phospholipase A2 activity is being assessed, the hydrophobic tail R2 must be connected to the remainder of the molecule by an ester linkage.
As used herein, "alkyl" by itself or as part of another substituent refers to a saturated or unsaturated branched, straight-chain or cyclic monovalent hydrocarbon radical having the stated number of carbon atoms (i.e., C1-C6 means one to six carbon atoms) that is derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl, cycloprop-1-en-1-yl; cycloprop-2-en-1-yl, prop-1-yn-1-yl, prop-2-yn-1-yl, etc.; butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, etc.; and the like. Where specific levels of saturation are intended, the nomenclature "alkanyl," "alkenyl" and/or "alkynyl" is used, as defined below. In some embodiments, the alkyl groups are (C1-C6) alkyl.
As used herein, "aryl" by itself or as part of another substituent refers to a monovalent aromatic hydrocarbon group having the stated number of carbon atoms (i.e., C5-C15 means from 5 to 15 carbon atoms) derived by the removal of one hydrogen atom from a single carbon atom of a parent aromatic ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene, and the like, as well as the various hydro isomers thereof. In some embodiments, the aryl group is (C5-C15) aryl, with (C5-C10) being even more preferred. Particularly preferred aryls are cyclopentadienyl, phenyl and naphthyl.
As used herein, "arylalkyl" by itself or as part of another substituent refers to an acyclic alkyl group in which one of the hydrogen atoms bonded to a carbon atom, typically a terminal or sp³ carbon atom, is replaced with an aryl group. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylakenyl and/or arylalkynyl is used. In some embodiments, the arylalkyl group is (C6-C21) arylalkyl, e.g., the alkanyl, alkenyl or alkynyl moiety of the arylalkyl group is (C1-C6) and the aryl moiety is (C5-C15). In some embodiments the arylalkyl group is (C6-C13), e.g., the alkanyl, alkenyl or alkynyl moiety of the arylalkyl group is (C1-C3) and the aryl moiety is (C5-C10).

In one embodiment of the invention, the R1 and R2 chains are selected from amongst the alkyl chains commonly known to comprise phospholipids such that the hydrophobic moiety of the labeled phospholipase substrate corresponds to the hydrophobic moiety of a naturally occurring phospholipid. Non-limiting examples of suitable n-alkyl chains include those derived from commonly occurring fatty acids, such as the following fatty acids: myristic acid (length - number of unsaturated C-C bonds: 14-0), palmitic acid (16-0), stearic acid (18-0), oleic acid (18-1 cisΔ⁹), linoleic acid (18-2 cisΔ^{9,12}), linonenic acid (18-3 cisΔ^{9,12,15}), arachidonic acid (20-4 cisΔ^{5,8,11,14}), eicosapentaenois acid (20-5 cisΔ^{5,8,11,14,17}).

In one embodiment of the invention, said anionic glycerophospholipid is selected in the group of phosphatidylinositol, phosphatidylglycerol, phosphatidylmethanol, phosphatidylserine, and phosphatidic acid.

According to the invention, the substrate may be labelled with a fluorophore on the terminal end of the sn-1 fatty acyl chain and/or on the terminal end of the sn-2 fatty acyl chain. In one embodiment of the invention, the substrate is labelled directly with the fluorophore. In another embodiment of the invention, the substrate is labelled with the fluorophore via a linker. Said linker is preferably an hydrophobic linker such as for example an alkyl chain.

In one embodiment of the invention, when phospholipase A1 activity is being assessed, the substrate is labelled with a fluorophore at least on the terminal end of the sn-1 fatty acyl chain.
In another embodiment of the invention, when phospholipase A2 activity is being assessed, the substrate is labelled with a fluorophore at least on the terminal end of the sn-2 fatty acyl chain.

According to the invention, the fluorophore may be any fluorophore having the following properties: it is capable of producing a detectable fluorescence signal in the assay medium, the fluorescence signal can be "self-quenched" and it is capable of fluorescing in an aqueous medium.
As used herein, "quench" refers to a reduction in the fluorescence intensity of a fluorescent group as measured at a specified wavelength, regardless of the mechanism by which the reduction is achieved. As specific examples, the quenching may be due to molecular collision, energy transfer such as FRET, a change in the fluorescence spectrum (color) of the fluorescent group or any other mechanism. The amount of the reduction is not critical and may vary over a broad range. The only requirement is that the reduction be measurable by the detection system being used. Thus, a fluorescence signal is "quenched" if its intensity at a specified wavelength is reduced by any measurable amount.
Without willing to be bound to a theory, the inventors state that, when the substrate packs into the membrane bilayer, the close proximity of the fluorophore, from neighboring phospholipids, causes the spectral properties to change relative to that of monomeric molecule (owing to exciplex formation or quenching). When the fluorophore fatty acid is liberated from the glycerol backbone owing to the phospholipase-catalyzed reaction, it is captured by the bovine serum albumine present in the aqueous phase. These features allow for a sensitive phospholipase assay by monitoring the fluorescence of monomeric, albumin-bound fluorophore fatty acid. This fluorimetric assay monitors product formation continuously and the sensitivity of the assay approaches that of conventional fixed time-point assays with radiolabeled phospholipids.
Examples of such fluorophore are xanthenes such as fluoresceins, rhodamines and rhodols, cyanines, phtalocyanines, squairanines, bodipy dyes, pyrene, anthracene, naphthalene, acridine, stilbene, indole or benzindole, oxazole or benzoxazole, thiazole or benzothiazole, carbocyanine, carbostyryl, prophyrin, salicylate, anthranilate, azulene, perylene, pyridine, quinoline, borapolyazaindacene, xanthene, oxazine or benzoxazine, carbazine, phenalenone, coumarin, benzofuran, or benzphenalenone.

Examples of suitable rhodamine dyes include, but are not limited to, rhodamine B, 5-carboxyrhodamine, rhodamine X (ROX), 4,7-dichlororhodamine X (dROX), rhodamine 6G (R6G), 4,7-dichlororhodamine 6G, rhodamine 110 (R110), 4,7-dichlororhodamine 110 (dR110), tetramethyl rhodamine (TAMRA) and 4,7-dichlorotetramethylrhodamine (dTAMRA).
Examples of fluorescein dyes include, but are not limited to, 4,7-dichlorofluoresceins, 5-carboxyfluorescein (5-FAM) and 6-carboxyfluorescein (6-FAM).
Examples of commercially available fluorescent dyes include, but are not limited to, 5-carboxyfluorescein-bis-(5-carboxymethoxy-2-nitrobenzyl) ether, -alanine-carboxamide, succinimidyl ester (CMNB-caged carboxyfluorescein, SE); 5-carboxyfluorescein, succinimidyl ester (5-FAM, SE); 5-(and-6)-carboxyfluorescein, succinimidyl ester (5(6)-FAM, SE); 5-(4,6-dichlorotriazinyl) aminofluorescein (5-DTAF); 6-(fluorescein-5-carboxamido)hexanoic acid, succinimidyl ester (5-SFX); 6-(fluorescein-5-(and-6)-carboxamido) hexanoic acid, succinimidyl ester (5(6)-SFX); 6-(fluorescein-5-(and-6)-carboxamido) hexanoic acid, succinimidyl ester (5(6)-SFX); fluorescein-5-EX, succinimidyl ester; fluorescein-5-isothiocyanate (FITC 'Isomer I'); fluorescein-5-isothiocyanate (FITC 'Isomer I'); fluorescein-5-isothiocyanate (FITC 'Isomer I'); fluorescein-6-isothiocyanate (FITC 'Isomer II'); Texas Red® sulfonyl chloride; Texas Red® sulfonyl chloride; Texas Red®-X, STP ester, sodium salt; Texas Red®-X, succinimidyl ester; Texas Red®-X, succinimidyl ester.
In one embodiment of the invention, said substrate is labelled with a pyrene such as benzo(d,e,f)phenanthrene, or BODIPY FL (4, 4-difluoro-5,7-dimethyl-4-bora-3a, 4a-diaza-s- indacene).

Phospholipids can be prepared using conventional synthetic methods. The synthesis of phospholipids is described in Phospholipids Handbook, G. Cevc, ed., Marcel Dekker (1993), G Hermanson, Bioconjugate Techniques, Academic Press (1996) and Subramanian et al. ARKIVOC VII:116-125 (2002)). For example, phospholipids can be prepared from the reaction of a 3-substituted phosphoglycero compound with selected fatty acid anhydrides. Alternatively, phospholipids can be extracted from natural sources (e.g. egg yolk, brain, or plant sources) or can be purchased commercially (e.g. from Sigma-Aldrich and Avanti Polar Lipids). Examples of suitable phosphoglycero compounds include glycero-3-phosphoethanolamine and glycerol-3-phosphoserine, either of which can be obtained commercially (e.g. from Sigma-Aldrich), or obtained by extraction from natural sources. Fatty acid anhydrides are prepared from fatty acids which in turn can be synthesized by conventional methods, extracted from natural sources, or purchased commercially.

In another embodiment of the invention, said substrate is labelled with a fluorophore on one of the sn-1 or sn-2 fatty acid chain and a quencher is present on the remaining sn-1 or sn-2 fatty acid chain.
Without willing to be bound to a theory, the inventors states that, when these phospholipids pack into the membrane bilayer, the close proximity of the two fluorophores, from neighboring phospholipids molecules and intramolecularly, causes the spectral properties to change relative to that of monomeric molecule (quenching). FRET (Förster resonance energy transfer) is a distance-dependent transfer of excited state energy from an initially excited donor to an acceptor, with the donor molecule typically emitting at shorter wavelengths that overlap with the absorption of an acceptor. FRET occurs when a donor (fluorophore) and an acceptor (another fluorophore or quencher) are within a specified distance, usually within 10-100 Å. The donor-acceptor distance at which the energy transfer is 50% is called the Förster radius (Ro). Within this distance, when a donor transfers its resonance energy to a quencher, a decrease in the donor fluorescence is seen. FRET efficiency falls dramatically as the donor-acceptor distance exceeds the Förster radius.
Examples of FRET donor acceptor couples that can be used to label sn-1 and sn-2 fatty acyl chain include, but are not limited to, 7-diethylamino-3((4-iodoacetyl)amino) (NBD) (donor) and N-(7-nitro-2-1,3-benzoxadiazol-4-yl) (acceptor) or 7-diethylamino-3((4' iodoacetyl)amino) (NBD) (donor) and 9-diethylamino-5H-benzo[alpha]phenoxazine-5-one (Nile Red) (acceptor).
An example of a substrate to be used for determining phospholipase A2 activity is a glycerophospholipid wherein NBD fluorophore is covalently attached at sn-1 position with nonhydrolysable ether linkage and Nile Red is attached at sn-2 position with ester bond. Another example of a substrate to be used for determining phospholipase A2 activity is a glycerophospholipid wherein a borapolyazaindacene fluorophore, preferably 4,4-difluoro-4-bora-3a-,4a-diaza-s-indacene (BODIPY), is covalently attached at sn-1 position with nonhydrolysable ether linkage and is attached at sn-2 position with ester bond.

An example of a substrate to be used for determining phospholipase A1 activity is a glycerophospholipid wherein a borapolyazaindacene fluorophore, preferably 4,4-difluoro-4-bora-3a-,4a-diaza-s-indacene (BODIPY), is covalently attached at sn-1 position with ester bond and is attached at sn-2 position with nonhydrolysable ether linkage.

In another embodiment of the invention, when phospholipase A1 activity is being assessed, the substrate is labelled with an unsubstituted or substituted pyrene as a fluorophore on the terminal end of the sn-2 fatty acyl chain and with a quencher on the sn-1 fatty acyl chain.
In another embodiment of the invention, when phospholipase A2 activity is being assessed, the substrate is labelled with an unsubstituted or substituted pyrene as a fluorophore on the terminal end of the sn-1 fatty acyl chain and with a quencher on the sn-2 fatty acyl chain. Although these substrates comprise unsubstituted or substituted pyrene as a fluorophore, they are not fluorescent as such fluorescence is quenched by the quencher. Under the influence of phospholipase A1 or A2, the quencher is cleaved off and spatially separated from the remaining substrate, thus allowing an increase in fluorescence intensity which is directly proportional to phospholipase A1 or A2 activity.
The pyrenes used as fluorophore present the advantages of a lower polarity, a great distance between the extinction wavelength and the emission wavelength, the ability to work in aqueous solutions and the formation of excimers at higher concentrations and simultaneous shift of the emission wavelength to greater wavelengths.
According to this embodiment, examples of quenchers include, but are not limited to, phenyl residues, which are substituted with one or more, in particular two, nitro groups, such as for example the 2,4-dinitrophenyl residue.
According to this embodiment, the fluorophore and the quencher are bound to the sn-1 and sn-2 fatty acyl chains via a linker.
In one embodiment, the linker used to connect the fluorophore to the sn-1 or sn-2 fatty acyl chains may be derived from an ether (R1-OR2)ₘ, wherein R1 and R2 are independently selected and are derived from a hydrocarbon having 1 to 12 carbon atoms, with m being an integer from 1 to 4. Preferably, R1 and R2 are (CH2)n, wherein n is an integer from 2 to 12. An example of preferred linker is (CH2)₄-O-(CH2)₁₀. Linkers comprising CH2 chains are especially favourable as they have particular metabolic stability.

In another embodiment, the linker used to connect the fluorophore to the sn-1 or sn-2 fatty acyl chains may be derived from a hydrocarbon R, which is preferably (CH2)ₒ, wherein o is an integer from 1 to 20, in particular from 2 to 6. These linkers are also metabolically stable.
In one embodiment, the linker used to connect the quencher to the sn-1 or sn-2 fatty acyl chains may be derived from C(O)-(CH2)ₚ or C(O)-(CH2)ₚ-NH, wherein p is an integer from 1 to 20, preferably from 2 to 6. Preferably, said linker is C(O)-(CH2)₅-NH. These linkers are particularly well-suited as they are metabolically stable.
The person skilled in the art will use conventional reactions and methods in synthetic organic chemistry for preparing the substrate of the invention.

Examples of fluorescently-labelled substrates that can be coated to a solid phase are the following: fluorescent glycerol based lipids commercialized by Aventi (Phosphatidylcholine (NBD), , Phosphatidic Acid (NBD), Phosphatidylethanolamine (NBD), Phosphatidylglycerol (NBD), Phosphatidylserine (NBD) ; fluorescent phospholipids commercialized by molecular probes (Phosphocholine BODIPY D-3793, D-3795, Phosphocholine BODIPY FL D-3792, D-3803 and D-3771, Phosphatidic acid BODIPY FL D-3805, Phosphoethanolamine BODIPY D-3813, Phosphocholine BODIPY D-3806, Phosphocholine DPH D-476, Phosphocholine NBD N-3786 and N-3787, Phosphocholine Perylene H-3790, Phosphocholine Pyrene H-361, H-3818, B-3781 and B-3782, Phosphoethanolamine Pyrene H-3784, Phosphoglycerol Pyrene H-3809 and Phosphomethanol Pyrene H-3810 and O-7703).

One object of the invention is a method for measuring a phospholipase A1 or A2 activity in a sample, said method comprising:
- contacting the sample with a solid phase coated with a fluorescently-labeled phospolipase A1 or A2 substrate as described here above under conditions effective to permit the phospholipase to cleave the fluorescently-labeled phospholipase substrate,
- detecting the fluorescence as a function of time.

In one embodiment of the invention, the sample is selected from urine, serum, plasma sample, whole blood, bronchoalveolar lavage fluid, sputum, synovial fluid, amniotic fluid, peritoneal fluid, cerebrospinal fluid, pleural fluid, pericardial fluid, and alveolar macrophages.
A "sample" in the context of the invention may be a cell culture sample or a biological sample isolated from a subject and can include, by way of example and not limitation, bodily fluids and/or tissue extracts such as homogenates or solubilized tissue obtained from a subject. Tissue extracts are obtained routinely from tissue biopsy and autopsy material.

In another embodiment of the invention, the method of the invention as described here above may also be used for measuring phospholipase A1 or A2 in a cell culture sample. Another object of the invention is also an improved method for measuring calcium-dependent phospholipase A1 and A2 activity in a sample.
A calcium-dependent phospholipase A2 is secreted phospholipase A2 (sPLA2).
A calcium-dependent phospholipase A1 is phosphatidylserine (PS)-specific PLA1 (PS-PLA1), membrane-associated phosphatidic acid (PA)-selective PLA1s (mPA-PLAlalpha and mPA-PLAlbeta), and endothelial lipase (EL).

Said method for measuring calcium-dependent phospholipase A1 or A2 activity in a sample, comprising the following steps:
a) contacting a labelled substrate with said sample in conditions where phospholipase A1 or A2 enzymatic activity is blocked,
b) reading the fluorescence emission overtime
c) adding a solution to initiate phospholipase A1 or A2 enzymatic activity
d) reading the fluorescence emission overtime
   wherein the labelled substrate is as described here above.

In another embodiment of the invention, step a) is performed in the presence of at least one divalent ions chelating agent.
Said divalent ions chelating agent includes, but is not limited to, diaminoethanetetraacetic acid (EDTA) or ethylene glycol tetraacetic acid (EGTA).

In one embodiment, said divalent ions chelating agent is ethylene glycol tetraacetic acid (EGTA).

In another embodiment of the invention, the concentration of the divalent ions chelating agent is from 1 mM to 20 mM, preferably from 2 mM to 10 mM, more preferably 3 mM to 6 mM.

In another embodiment of the invention, the fluorescence emission is read overtime: for example from 30s to 5 min, preferably from 60s to 3 min, more preferably from 90s to 2 min.
The fluorescence emission is read at a wave length determined by the fluorophore used.

In another embodiment of the invention, the solution that initiates phospholipase A1 or A2 enzymatic activity comprises an excess of Ca2+ divalent ions.
Examples of divalent ions include, but are not limited to, calcium chloride (CaCl2), calcium bromide (CaBr2), calcium sulfate (CaSO2) calcium fluoride (CaF2), calcium iodate (CaI2O6), or calcium iodide (CaI). Preferably, said Ca2+ ions are CaCl2.

In another embodiment of the invention, the concentration of Ca2+ ions in the solution is from 1 to 10 mM, preferably from 2 mM to 8 mM, more preferably from 4 mM to 7 mM.

In one embodiment of the invention, the solution that initiates phospholipase A1 or A2 enzymatic activity is added manually. In another embodiment of the invention, the solution that initiates phospholipase A1 or A2 enzymatic activity is added automatically.

In another embodiment of the invention, the pH of the reaction is from 6 to 10, preferably from 7 to 9, more preferably from 8 to 9.

Another object of the invention is a kit comprising:
- a first container comprising a labelled substrate, wherein said labelled substrate is stabilised on a solid phase as described here above,
- a second container comprising an assay buffer comprising at least a buffer solution.

In one embodiment, said kit comprises:
- a first container comprising a labelled substrate, wherein said labelled substrate is stabilised on a solid phase as described here above,
- a second container comprising an assay buffer comprising at least a buffer solution, one or more divalent ions chelating agent, and bovine serum albumin,
- a third container comprising a solution that initiates phospholipase A1 or A2 enzymatic activity comprising Ca2+ divalent ions.
In one embodiment of the invention, the labelled substrate stabilised on a solid phase is in suspension, in an aqueous solution, in a dried form or in a lyophilised form.

In one embodiment of the invention, the assay buffer comprises a buffer solution such as Acetate, ADA, AMP, AMPD, AMPSO, Bicin, Bis-Tris-Propane, Glycylglycine, HEPES, HEPPS, HEPPSO, Phosphate, POPSO, TAPS, Taurine, Tricine, and Triethanolamine.

In one embodiment of the invention, the assay buffer comprises divalent ions chelating agent including, but not limited to, diaminoethanetetraacetic acid (EDTA) or ethylene glycol tetraacetic acid (EGTA). Preferably, said divalent ions chelating agent is ethylene glycol tetraacetic acid (EGTA).
In another embodiment of the invention, the concentration of the divalent ions chelating agent is from 1 mM to 20 mM, preferably from 2 mM to 10 mM, more preferably 3 mM to 6 mM.

In one embodiment of the invention, the assay buffer comprises bovine serum albumin, preferably fatty acid free bovine serum albumin.
In one embodiment, the range of bovine serum albumin present in the assay buffer is from 0.05% to 10%, preferably from 0.075% to 5%, more preferably from 0.1% to 0.5%.

In another embodiment of the invention, the assay buffer further comprises NaCl. In one embodiment, the concentration of NaCl present in the assay buffer is from 10 mM to 500 mM, preferably from 100 mM to 250 mM, more preferably from 130 mM to 170 mM.

In one embodiment of the invention, the kit further comprises or is provided with at least one calibrator and/or at least one control sample.

A calibrator for use in a method for measuring PLA1 activity can be any PLA1.
One example of PLA1 calibrator is the Human pancreatic Lipase (from pancreatic juice) BCR693 from Sigma Aldrich. The calibrator sample is in general a sample of the same nature than the sample to be tested, spiked with the PLA1 enzyme.

A calibrator for use in a method for measuring PLA2 activity can be any PLA2.
One example of sPLA2 calibrator is preferably sPLA2 extracted from bee venom (bv-sPLA2) spiked in human sample of the same nature than the sample to be tested. For example, if the sample to be tested is a plasma or serum sample, the calibrator will be a sample comprising sPLA2 spiked in human plasma or serum.
Said calibrator may be provided at different concentrations: for example 0.15 mg/ml, 0.2 mg/ml, 0.25 mg/ml, 0.3 mg/ml, 0.35 mg/ml and 0.4 mg/ml.

Control samples may be negative and/or positive controls.
Positive control is for example a sample having a known PLA1 or PLA2 activity or a sample comprising a known concentration of PLA1 or PLA2. For example a positive control to be used in the method for measuring sPLA2 activity is a sample comprising a known concentration of bv-sPLA2. As for the calibrators, the samples used for positive or negative controls are of the same nature than the sample to be tested.
Preferably, the concentration of PLA1 or PLA2 present in the positive sample is different from the concentrations of PLA1 or PLA2 present in the calibrators. For example, the concentration of sPLA2 in the positive control may be 0.275 mg/ml.
Negative control is for example a sample from substantially healthy donor. A substantially healthy donor as used herein refers to a donor that has not been diagnosed for a PLA1 or PLA2 activity-linked condition or that presents no symptom of a PLA1 or PLA2 activity-linked condition.

Another object of the invention is a method of identifying a subject having or at risk of having or developing a sPLA2 activity-linked condition, comprising measuring the sPLA2 enzymatic activity in a sample from the subject according to the method of the invention as described here above.
A "subject" in the context of the present invention is preferably a mammal. The mammal can be a human, non-human primate, mouse, rat, dog, cat, horse, or cow, but are not limited to these examples.

In one embodiment, said sPLA2 activity-linked condition includes, but is not limited to, inflammatory diseases, cancer, sepsis, burnt offering, severe surgery or other injuries with severe wound areas, diabetic shock, acute liver failure, pancreatitis, neurodegenerative diseases, autoimmune diseases e. g. SLE, osteo arthritis, rheumatoid arhritis, multiple sclerosis, myasthenia gravis, Graves'disease, psoriasis vulgaris, dilated cardiomyopathy, diabetes mellitus, Bechterew, inflammatory bile disease, ulcerative colitis, Crohn's disease, idiopathic thrombocytopenia purpura (ITP), plastic anemia, idiopathic dilated cardiomyopathy (IDM), autoimmune thyroiditis, Goodpastures'disease, arterial and venous chronic inflammation.

In another embodiment, said sPLA2 activity-linked condition is a cardiovascular disease and/or a cardiovascular event.
Said cardiovascular disease and/or cardiovascular event includes, but is not limited to, Metabolic Syndrome, Syndrome X, atherosclerosis, atherothrombosis, coronary artery disease, stable and unstable angina pectoris, stroke, diseases of the aorta and its branches (such as aortic stenosis, thrombosis or aortic aneurysm), peripheral artery disease, peripheral vascular disease, cerebrovascular disease, and any acute ischemic cardiovascular event.

Recent published studies (see references 1-9) have demonstrated an increased risk of cardiovascular disease (CVD) associated with sPLA2 activity values in the second and third tertiles (upper 67% of the studied populations) vs. the first tertile (lower 33% of the studied populations). Therefore, a more conservative approach for identifying individuals with a significant increased risk for CVD attributable to sPLA2 may be the 50th percentile value of the population.
In the current sPLA2 Activity test, this threshold value corresponds to 39.7 U/mL and 33.5 U/mL for female and male, respectively.

Another object of the invention is a method of identifying a subject having or at risk of having or developing a PLA1 activity-linked condition, comprising measuring the PLA1 enzymatic activity in a sample from the subject according to the method of the invention as described here above.
A "subject" in the context of the present invention is preferably a mammal. The mammal can be a human, non-human primate, mouse, rat, dog, cat, horse, or cow, but are not limited to these examples.

In one embodiment, said PLA1 activity-linked condition includes, but is not limited to, acid lipase disease, Wolman's disease or Cholesteryl ester storage disease (CESD).

The invention will be more fully understood upon consideration of the following non-limiting examples.

### BRIEF DESCRIPTION OF THE FIGURES

**Figure 1****:** Phospholipases sites of action in the hydrolysis of glycerophospholipids
**Figure 2****:** Background Fluorescence emission at 405 nm versus phospholipid-coated beads molecular coverage.
**Figure 3****:** sPLA2 Activity (slope at the origin) versus phospholipid-coated beads molecular coverage.
**Figure 4****:** Progress curves obtained after addition of increasing amount of bv-sPLA2 to phospholipid-coated beads.
**Figure 5****:** Progress curves obtained after addition of 66 fmol of bv-sPLA2 to phospholipid-coated beads.
**Figure 6****:** Fluorescence at 405 nm (mean +/- SD) of phospholipid-coted latex beads in the presence of different amounts of BSA.
**Figure 7****:** Triglycerides interference in sPLA2 activity measurement according to the method of the invention
**Figure 8****:** Hemoglobin interference in sPLA2 activity measurement according to the method of the invention
**Figure 9****:** Bilirubin interference in sPLA2 activity measurement according to the method of the invention
**Figure 10****:** Progress curves after addition of 30 µL of human serum sample to 1 - hexadecanoyl-2-(1 -pyrenedecanoyl)-sn-glycero-3-phosphomethanol liposomes.
**Figure 11****:** bv-sPLA2 Standard Curve using published method
**Figure 12****:** bv-sPLA2 Standard Curve using the method of the invention
**Figure 13****:** Cumulative percent distribution of sPLA2 activity in male and female ages 40-59 years.

### EXAMPLES

### 1- Preparation of phospholipid-coated latex beads according to the method of the invention

### Material and Reagents

β-py-C10-phosphoglycerol (β-py-C10-PG) was from Molecular Probes/Invitrogen. ∅ 4.15 µm polystyrene microspheres (beads) were purchased from Merck/Estapor. All other reagents were from Sigma-Aldricht.

### Experimental Protocol

All the steps were performed in glassware, with no direct exposure to light.
β -py-C10-phosphoglycerol was solubilized using sonication in 10% methanol/90% Chloroform (v/v) in glass. The exact molarity of the β-py-C10-phosphoglycerol was determined with the help of a standard curve by absorbance at 342 nm through a five points Standard Curve (1/100 and 1/1600 dilution in Methanol, Cascade Dilution, factor 2) prepared from the sonicated stock solution with the blank defined as 1/100 dilution of the 10% methanol/90% Chloroform (v/v) solution in methanol.
10% bead suspension in methanol was prepared by dilution (1:5 v/v) with methanol, followed by sonication. The coating Solution was prepared by adding β-py-C10-phosphoglycerol (from the Stock Solution) in methanol. The Coating Solution was added to the beads methanol suspension (1:1 v/v) in a 50 mL glass tube and then solvent was evaporated at 37°C under gentle shaking (400 rpm) for 8 h. Beads were then washed three times by adding 150 mM NaCl to the dry pellets of beads/phospholipids, followed by sonication. Beads were recovered by centrifugation and supernatants kept for yield determination by absorbance measurement at 342 nm (blank 150 mM NaCl). Absorbances of washing supernants at 342 nm were corrected by applying a Methanol/Nacl ratio. This ration was defined as OD 342 nm of a 1/100 dilution of the Coating Solution in methanol divided by OD 342 nm of a 1/100 dilution of the Coating Solution in 150 mM NaCl.
Washings steps were repeated until absorbance at 342 nm measured in supernant is under 4% of the sum of the absorbances measured in all supernants. Yield of coating (typically 75 to 85%) was determined by subtracting the amount of phospholipid found in all washing supernatants to the initial stock solution put in presence of beads. Molecular coverage was defined as the number of molecules per unit area, expressed in nm². Beads were stored at 4°C, in glass, protected from light, in aliquot of 1 mL.

### 2- Influence of the molecular coverage of phospholipid-coated beads in sPLA2 activity assay

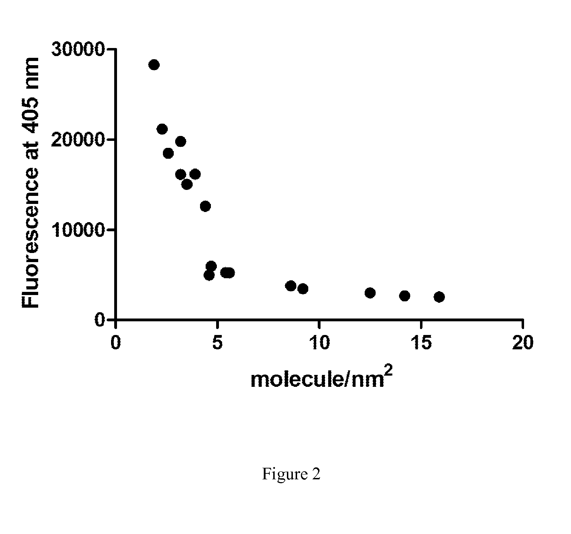

10 µl of 1% beads suspension with different molecular coverage were added to 190 µl of 10 mM Tris-HCl pH 8.5, 0.5% BSA, 6 mM EGTA and 100 mM NaCl and fluorescence emission at 405 nm with excitation at 355 nm was recorded for 180 sec in a Fluostar Optima fluorimeter equipped with a stirring device and thermostat ruled at 30°C. Fluorescence signal (arbitrary unit) was then normalized against the number of mole of β-py-C 10-phosphoglycerol added in the assay. Representative examples of the results of this analysis are presented in figure 2 and in the table 1:

**Table 1**

| Sample | Molecular Coverage (molecule/nm²) | Background (FU/nmol) |
|---|---|---|
| A | 1.9 | 28275.0 |
| B | 3.2 | 19778.0 |
| C | 3.9 | 16157.7 |
| D | 2.6 | 18488.0 |
| E | 4.7 | 5960.8 |
| F | 5.6 | 5240.3 |
| G | 2.3 | 21169.7 |
| H | 3.2 | 16149.7 |
| I | 3.5 | 15032.7 |
| J | 4.4 | 12621.4 |
| K | 4.6 | 4987.2 |
| L | 5.4 | 5272.2 |
| M | 8.6 | 3776.3 |
| N | 9.2 | 3457.5 |
| O | 12.5 | 3009.2 |
| P | 14.2 | 2699.5 |
| Q | 15.9 | 2566.2 |

As presented in figure 2, normalized background dropped with increasing molecular coverage, with a minimum around 2500-3000 FU for molecular coverage in the range of 7-15 molecule/nm².

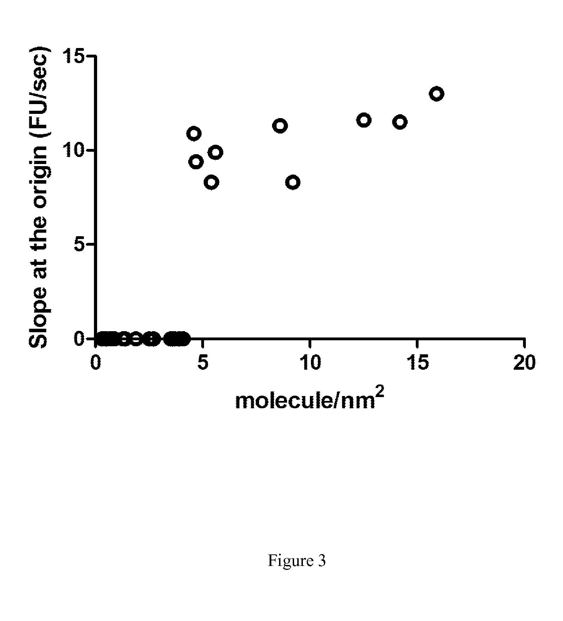

660 fmol of purified bee-venom sPLA2 (bv-sPLA2, SIGMA-ALDRICH) spiked in 30 µl of human serum were mixed with 10 µl of 1% beads suspension added to 190 µl of 10 mM Tris-HCl pH 8.5, 0.5% BSA, 5 mM CaCl2 and 100 mM NaCl. Fluorescence emission at 405 nm with excitation at 355 nm was recorded for 180 sec in a Fluostar Optima fluorimeter equipped with a stirring device and thermostat ruled at 30°C, and slope at the origin (expressed in FU/sec) were extracted from progress curves. Representative examples of the results of the assays comparing different molecular coverage are presented in figure 3 and in the table 2:

**Table 2**

| Beads Lot | Bead Diameter (µm) | Molecular Coverage (molecule/nm2) | Slope at the origin (FU/sec) |
|---|---|---|---|
| 1 | 2.2 | 0.3 | 0.0 |
| 2 | 2.2 | 0.4 | 0.0 |
| 3 | 4.2 | 0.5 | 0.0 |
| 4 | 2.2 | 0.7 | 0.0 |
| 5 | 4.2 | 0.8 | 0.0 |
| 6 | 2.2 | 0.8 | 0.0 |
| 7 | 2.2 | 0.9 | 0.0 |
| 8 | 2.2 | 1.3 | 0.0 |
| 9 | 2.2 | 1.4 | 0.0 |
| 10 | 4.2 | 1.9 | 0.0 |
| 11 | 4.2 | 2.5 | 0.0 |
| 12 | 4.2 | 2.7 | 0.0 |
| 13 | 4.2 | 3.5 | 0.0 |
| 14 | 2.2 | 3.6 | 0.0 |
| 15 | 2.2 | 3.7 | 0.0 |
| 16 | 4.2 | 3.9 | 0.0 |
| 17 | 2.2 | 4.1 | 0.0 |
| 18 | 4.2 | 4.6 | 10.9 |
| 19 | 4.2 | 4.7 | 9.4 |
| 20 | 4.2 | 5.4 | 8.3 |
| 21 | 4.2 | 5.6 | 9.9 |
| 22 | 4.2 | 8.6 | 11.3 |
| 23 | 4.2 | 9.2 | 8.3 |
| 24 | 4.2 | 12.5 | 11.6 |
| 25 | 4.2 | 14.2 | 11.5 |
| 26 | 4.2 | 15.9 | 13.0 |

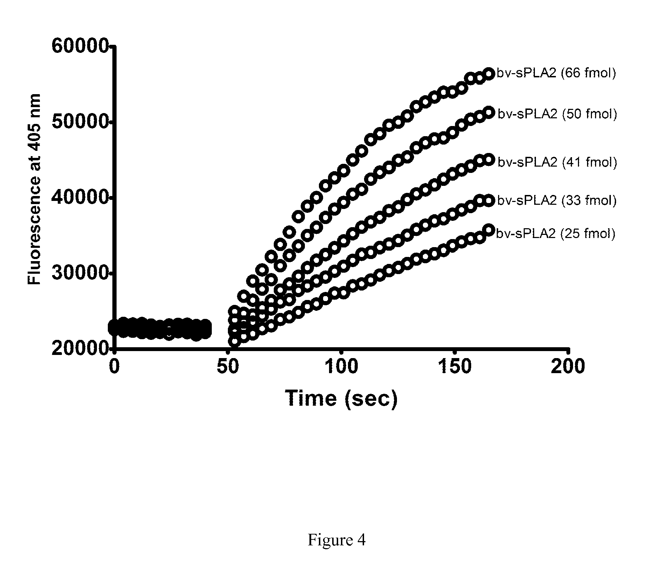

As presented in figure 3, no sPLA2 activity was observed using phospholipid-coated beads with molecular coverage below 4 molecule/nm². This observation may be explained by the fact that phospholipid is not organized as a proper bilayer at that concentration, and thus not recognized by the enzyme.
Activity is then observed when molecular coverage reached 4 molecule/nm² and increased slightly with increasing coverage.
The better compromise between coating yield (data not schown), background and signal in the sPLA2 activity assay was centered around 10 molecule/nm². 5 different amounts of purified bv-sPLA2 (25, 33, 41, 50 and 66 fmol) spiked in 30 µl of human serum were mixed with 10 µl of 1% beads suspension (molecular coverage 91 molecule/nm²) added to 190 µl of 10 mM Tris-HCl pH 8.5, 0.5% BSA, 5 mM EGTA and 100 mM NaCl. Fluorescence emission at 405 nm with excitation at 355 nm was recorded for 50 sec in a Fluostar Optima fluorimeter equipped with a stirring device and thermostat ruled at 30°C. At 53 sec, 20 µl of a 150 mM CaCl2 solution was added to the mixture and fluorescence emission at 405 nm with excitation at 355 nm was recorded for additional 125 sec.
A representative example of the progress curves obtained during these assays is presented in figure 4.

As presented in figure 4, progress curves obtained after addition of increasing amount of bv-sPLA2 to phospholipid-coated beads display higher slopes demonstrating that the assay is quantitative in this range of concentration.

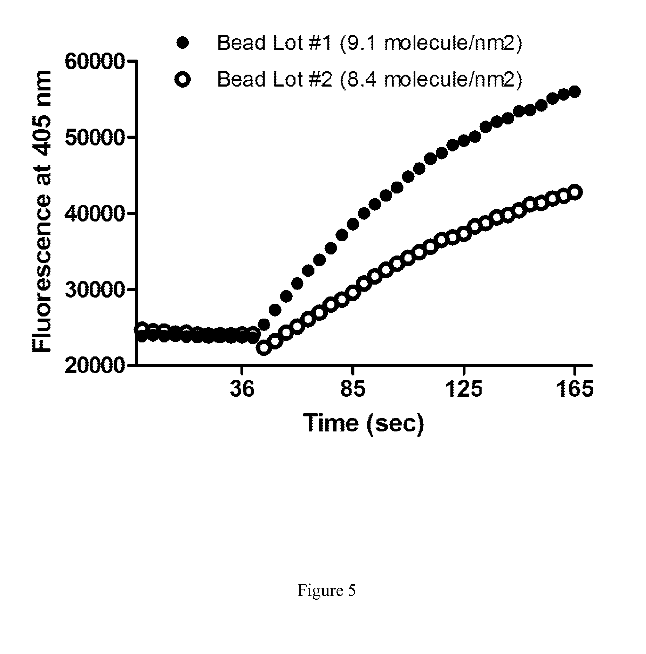

66 fmol of purified bv-sPLA2 spiked in 30 µl of human serum were mixed with 10 µl of 1% beads suspension (molecular coverage 8.4 and 9.1 molecule/nm², respectively) added to 190 µl of 10 mM Tris-HCl pH 8.5, 0.5% BSA, 5 mM EGTA and 100 mM NaCl. Fluorescence emission at 405 nm with excitation at 355 nm was recorded for 50 sec in a Fluostar Optima fluorimeter equipped with a stirring device and thermostat ruled at 30°C. At 53 sec, 20 µl of a 150 mM CaCl2 solution was added to the mixture and fluorescence emission at 405 nm with excitation at 355 nm was recorded for additional 125 sec.
A representative example of the progress curves obtained during these assays is presented in figure 5.
As presented in figure 5, sPLA2 activity (illustrated here as the slope at the origin) is directly influenced by the molecular coverage of the phospholipid-coated beads used in the assay. Preparation mode including washings, determination of molecular coverage and amounts of phospholipid-coated beads added to the assay are then crucial for sensitive sPLA2 assay.

### 3- Influence of BSA in sPLA2 activity assay

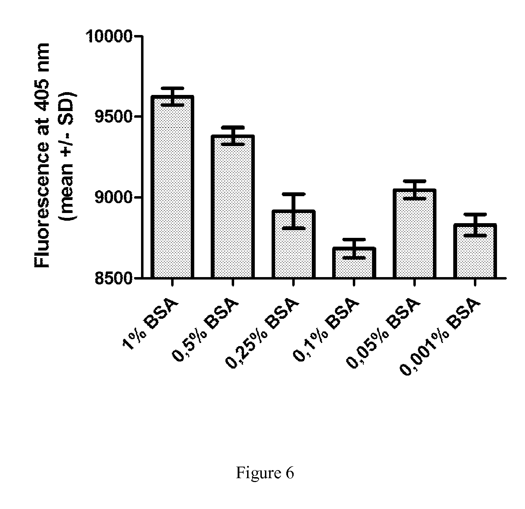

10 µl of 1% beads suspension were added to 190 µl of 10 mM Tris-HCl pH 8.5, 6 mM EGTA and 100 mM NaCl and different BSA concentrations (1%, 0.5%, 0.25%, 0.1%, 0.05% and 0.001%, respectively). Fluorescence emission at 405 nm with excitation at 355 nm was recorded for 100 sec in a Fluostar Optima fluorimeter equipped with a stirring device and thermostat ruled at 30°C. Representative example of this analysis fluorescence mean is presented in figure 6.

As illustrated in figure 6, concentrations of BSA have major influence in fluorescence emission in the method of invention. Reorganization and/or cross-linking of the phospholipid-coated beads complex by BSA may explain those observations.

### 4- Performance characteristics of the method of the invention

### Material and Reagents

β-py-C10-phosphoglycerol (β-py-C10-PG) was from Molecular Probes/Invitrogen. ∅ 4.15 µm polystyrene microspheres (beads) were purchased from Merck/Estapor. All other reagents were from Sigma-Aldricht.

### Experimental Protocol

sPLA2 activity was measured according to the method of the invention. 30 µl of serum sample were added to 1 -hexadecanoyl-2-(1 -pyrenedecanoyl)-sn-glycero-3-phosphoglycerol stabilized on latex beads (10 µL of 1% beads suspension) in the presence of 200 µl of buffer (10 mM Tris-HCl pH 8.5. 0.5% BSA. 6mM EGTA and 100 mM NaCl) at 30°C in a 96-wells plate.
Background fluorescence emission was recorded at 405 nm with excitation at 355 nm for 90 sec in Fluostar Optima fluorimeter equipped with a stirring device and thermostat ruled at 30°C. The reaction was initiated by the injection of 20 µl of a CaCl2 solution (55 mM) automatically performed by the fluorimeter. Fluorescence emission at 405 nm with excitation at 355 nm after CaCl2 injection was recorded for 90 sec. Obtained progress curve was fitted using nonlinear regression. After subtraction of background level, final point at 1 min was determined from the obtained fitted curve equation. A standard calibration curve was obtained by plotting the final point at 1 min obtained for each of six sPLA2 Calibrators on the y-axis versus the sPLA2 concentration on the x-axis and final points at 1 min of each sample were then normalized using the standard calibration curve. sPLA2 activity are expressed as U/ml by converting the normalized fluorescence values into quantity (in nmol) of product liberated in one min. All samples were tested in duplicate

### 4.1-Assay Dynamic Range

Assay dynamic range was determined from assays of plasma samples from human subjects spiked with known amount of purified bee venom sPLA2 (bv-sPLA2).
Assay linearity: > 0.99 over the calibration range (quadratic polynomial fit). Linear response was determined from serially diluted bv-sPLA2 protein spiked in plasma assayed for sPLA2 activity.
Assay sensitivity:
   The minimum detection limit is 10 U/mL (determined from the lowest concentration of bv-sPLA2 enzyme that can be reliably measured in the linear range of the assay. i. e 0.025 pmol).
   The maximum detection limit is 250 U/mL (determined from the highest concentration of bv-sPLA2 enzyme that can be reliably measured in the linear range of the assay. i. e 0.5 pmol).

### 4.2-Assay Repeatability (intra-assay variability)

Intra assay variability was evaluated by the average %CV calculated from % CV of 8 plasma samples from human subjects distributed throughout the calibration range of the assay run 10 times on the same assay plate using a single lot of reagents on the same day. Intra-assay CV for individual for individual human plasma samples ranged from 4.61 to 13.59% with an average intra-assay CV of 8.46% as shown in Table 3.

**Table 3**

| **Sample** | **Replicate** | | | | | | | | | | | |
|---|---|---|---|---|---|---|---|---|---|---|---|---|
| | **1** | **2** | **3** | **4** | **5** | **6** | **7** | **8** | **9** | **10** | **Mean** | **CV** |
| A | 89.70 | 84.26 | 91.85 | 96.20 | 94.31 | 94.45 | 96.45 | 91.27 | 101.68 | 92.78 | 100.23 | **4.61%** |
| B | 112.02 | 95.99 | 106.73 | 121.36 | 97.34 | 107.90 | 108.32 | 102.31 | 115.42 | 104.21 | 129.14 | **6.05%** |
| C | 146.21 | 131.17 | 143.83 | 160.86 | 133.59 | 147.24 | 159.06 | 146.55 | 170.85 | 171.79 | 108.39 | **13.02%** |
| D | 66.57 | 51.38 | 57.83 | 75.95 | 62.97 | 68.06 | 61.95 | 64.20 | 68.86 | 78.87 | 94.51 | **8.54%** |
| E | 116.80 | 106.72 | 129.91 | 127.69 | 116.01 | 126.52 | 118.02 | 122.54 | 133.83 | 135.53 | 145.83 | **6.19%** |
| F | 175.13 | 149.96 | 161.83 | 170.30 | 166.13 | 163.17 | 169.36 | 177.65 | 170.25 | 179.34 | 144.31 | **6.00%** |
| G | 115.29 | 108.73 | 118.17 | 129.91 | 117.71 | 118.37 | 107.29 | 121.26 | 134.79 | 131.49 | 95.82 | **9.66%** |
| H | 63.23 | 55.19 | 64.08 | 81.73 | 73.77 | 67.79 | 60.36 | 68.79 | 82.71 | / | 68.63 | **13.59%** |
| **Mean CV** | | | | | | | | | | | | **8.46%** |

### 4.3-Assay Precision (inter-assay variability)

### Inter-assay variability

Intra assay variability was evaluated by the average % CV calculated from % CV of 8 plasma samples from human subjects run by two operators on three different days on two different fluorimeters using two different batches of assay substrate and reagents (see table 4).
Total inter-assay CV for individual human plasma samples ranged from 1.05 to 13.08% with an average inter-assay CV of 5.24%.

**Table 4**

| **Sample** | **Day 1** | | **Day 2** | | **Day 3** | | | |
|---|---|---|---|---|---|---|---|---|
| | **Operator 1** | **Operator 2** | **Operator 1** | **Operator 2** | **Operator 1** | **Operator 2** | **Mean** | **CV** |
| A | 211.6 | 207.2 | 204.2 | 210.9 | 202.4 | 215.9 | 208.7 | **2.43%** |
| B | 118.2 | 120.5 | 105.6 | 102.3 | 116.1 | 118.6 | 113.6 | **6.71%** |
| C | 149.9 | 149.2 | 148.3 | 149.2 | 147.2 | 144.2 | 148.0 | **1.41%** |
| D | 84.2 | 92.3 | 86.0 | 77.8 | 86.2 | 97.1 | 87.3 | **7.66%** |
| E | 71.1 | 83.2 | 68.6 | 58.4 | 80.1 | 82.0 | 73.9 | **13.08%** |
| F | 75.3 | 78.5 | 69.3 | 70.2 | 71.5 | 62.6 | 71.2 | **7.63%** |
| G | 184.0 | 183.1 | 188.9 | 180.2 | 187.9 | 181.1 | 184.2 | **1.92%** |
| H | 233.4 | 237.3 | 237.2 | 236.8 | 238.7 | 232.4 | 235.9 | **1.05%** |
| **Mean CV** | | | | | | | | **5.24%** |

### Repeatability study:

In a repeatability study conducted with a set of 63 plasma samples from human subjects, results determined on one day were compared to the mean results from two successive measurements performed on another day.
The sPLA2 activity levels of the samples ranged from 22.1 to 213 U/mL and the mean %CV between replicates was 5.1 %.
In linear regression analysis, first measurements were highly correlated to duplicate mean results: correlation coefficient r=0.987 (slope 0.973 and intercept 2.1 U/mL).

### 4.4-Freeze/thaw Effect

Four plasma samples were analyzed after each of four freeze/thaw cycles (-80°C to 4°C). No definitive trend in sPLA2 activity values was observed; indicating samples may be frozen and thawed four times as shown in Table 5.

**Table 5**

| **Freeze/Thaw cycles** | **Sample 1** | **Sample 2** | **Sample 3** | **Sample 4** |
|---|---|---|---|---|
| 1 | 58.66 | 29.37 | 134.80 | 31.60 |
| 2 | 56.09 | 25.51 | 121.68 | 28.53 |
| 3 | 55.48 | 28.40 | 131.42 | 22.70 |
| 4 | 61.50 | 30.10 | 126.06 | 21.16 |

### 4.5-Sample stability study:

Two freshly collected human plasma samples were stored at bench-top (room temperature) and 4°C and assayed after 1h, 2h, 3h, 4h, 6h, 72h and 18 days.
No definitive trend in sPLA2 activity values was observed over the 1h-72h period, indicating samples are highly stable over that period of time, as shown in Table 6.

**Table 6**

| **Sample/time** | **1h** | **2h** | **3h** | **4h** | **6h** | **72h** | **18 days** |
|---|---|---|---|---|---|---|---|
| A at room temp | 279.59 | 277.12 | 292.36 | 293.99 | 275.18 | 276.37 | 261.68 |
| A at 4°C | 271.56 | 264.65 | 281.07 | 277.65 | 282.37 | 268.35 | 219.45 |
| B at room temp | 224.32 | 226.65 | 221.62 | 250.19 | 242.43 | 215.60 | 195.01 |
| B at 40°C | 221.28 | 206.63 | 222.52 | 222.29 | 222.32 | 201.64 | 220.63 |

### 4.6-Preferred matrix

Blood samples from 10 healthy donors were collected on dry, heparin, citrate and EDTA sampling tubes on the same day, processed following standard procedures and stored at - 80°C.
A trend was observed in favor of EDTA plasma and serum, indicating that these two sampling tubes should be preferred when available (table 7).

**Table 7**

| **Donor ID** | **Serum** | **Heparin plasma** | **Citrate plasma** | **EDTA plasma** |
|---|---|---|---|---|
| 1 | 51.12 | 40.90 | 40.45 | 57.16 |
| 2 | 29.15 | 26.99 | 32.26 | 32.92 |
| 3 | 39.28 | 34.49 | 35.93 | 43.73 |
| 4 | 34.47 | 35.00 | 29.18 | 40.47 |
| 7 | 46.81 | 42.58 | 42.03 | 54.06 |
| 8 | 48.57 | 38.16 | 45.11 | 62.98 |
| 9 | | 37.61 | 39.42 | 48.64 |
| 10 | 40.19 | 44.01 | 43.78 | 55.90 |

### 4.7-Interfering Substances

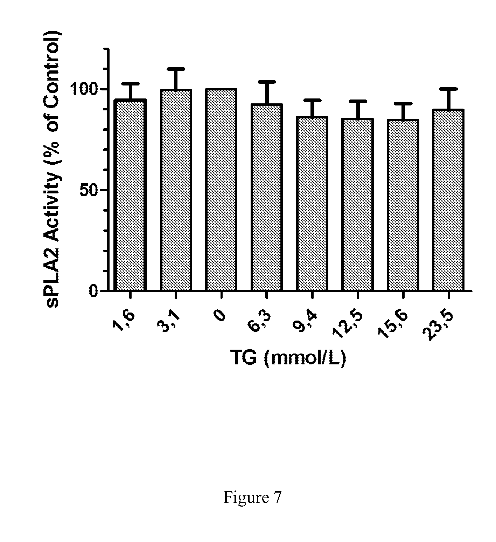

Possible interference by hemolysis icteria or lipemia was analyzed by adding hemoglobin, bilirubin and triglycerides to normal serum samples.
Samples with increased triglyceride showed no interference with triglyceride as high as 23 mmol/L (see figure 7).

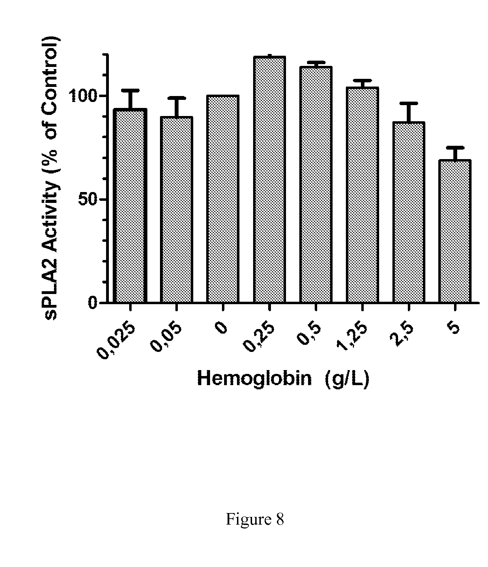

As the absorption wave-length of hemoglobin partially overlaps those of the fluorochrome used in the assay. Samples with increased hemoglobin showed partial interference with hemoglobin starting at 2 g/L (see figure 8).

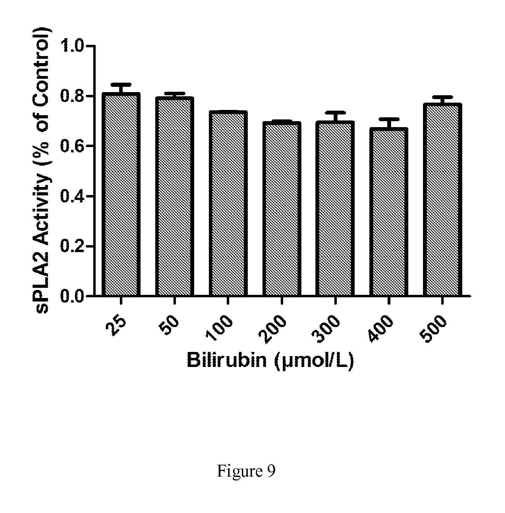

Samples with increased bilirubin showed no measurable interference with bilirubin concentration as high as 500 µmol/L (see figure 9).

### 5- Comparison between the method of the invention and previous published methods

### Material and Reagents

1 -hexadecanoyl-2-(1 -pyrenedecanoyl)-sn-glycero-3-phosphomethanol was from Molecular Probes/Invitrogen. All other reagents were from Sigma-Aldricht.

### Experimental Protocol

sPLA2 activity was measured according to the following published protocol:
30 µl of serum sample were added to 5 mmol of 1 -hexadecanoyl-2-(1 -pyrenedecanoyl)-sn-glycero-3-phosphomethanol liposomes in the presence of 10 mM Tris-HCl pH 8.7, 0.1% BSA, 10 mM CaCl2. Fluorescence emission was recorded at 405 nm with excitation at 355 nm for 60 sec in Fluostar Optima fluorimeter equipped with a stirring device and thermostat ruled at 30°C.
100% hydrolysis of the substrate is obtained with 0.1 U of bee venom PLA2 during one minute, the value of the fluorescence at the end of the one minute reaction (Fmax) thus corresponds to an activity of 2 nmoles/min (Vmax). The activity (A) of the sample (expressed in nmol/ml/min) is given by the following formula: V max .F A = E.Fmax. The samples are diluted when substrate hydrolysis is above 50%. The hydrolysis of substrate in the absence of plasma is used as negative control and deduced from PLA2 activity.

### 5.1-Specificity

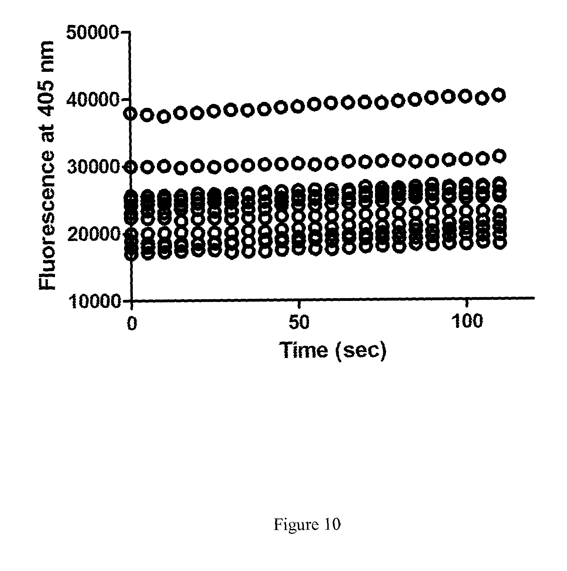

Fluorescence emission was recorded over time at 405 nm with excitation at 355 nm after addition of 30 µL human serum sample to 5 mmol of 1 -hexadecanoyl-2-(1 - pyrenedecanoyl)-sn-glycero-3-phosphomethanol liposomes in the presence of 10 mM Tris-HCl pH 8.7, 0.1% BSA, 10 mM CaCl2. As illustrated in figure 10, these 11 human serum samples displayed no or very little sPLA2 activity, as demonstrated by very slow initial velocities.
However, background fluorescence emissions in the first seconds of the progress curves of these 11 human samples are very different, ranging from 16000 to 39000 fluorescence units.

These data are the clear demonstration that unknown factors present in human serum sample can greatly influence the structure of the liposomes used as substrate in phospholipase enzymatic assays and therefore influence the measurement of phospholipase activity.

The method of the invention is more specific than previous published methods, as the level of background is:
- Measured and subtracted from the level of fluorescence signal.
- Better measured as background and signal are measured in the same reaction mixture, in the same well, in the presence of the same sample.
- Highly reduced due to stabilisation of phospholipid substrate on solid phase.

### 5.2-Sensitivity and linearity

The lower limit of detection, defined as the mean of background +/- 3 SD is 10 U/mL for the method of the invention. The lower limits of detection of existing methods, defined similarly (mean of background +/- 3 SD) and expressed in the same units, are in the range of 200 U/mL (data not shown).
The method of invention is much more sensitive than previous published methods. The gain in sensitivity of the method of the invention is 10 to 20 fold, compared to existing methods, mainly due to as the automatic injection enables the detection of fluorescence emission about 10-20 sec earlier than previous published methods, in which the enzymatic reaction is initiated by the addition of the sample. This major improvement leads to the access to earlier points of the kinetic progress curve where initial velocity is faster.

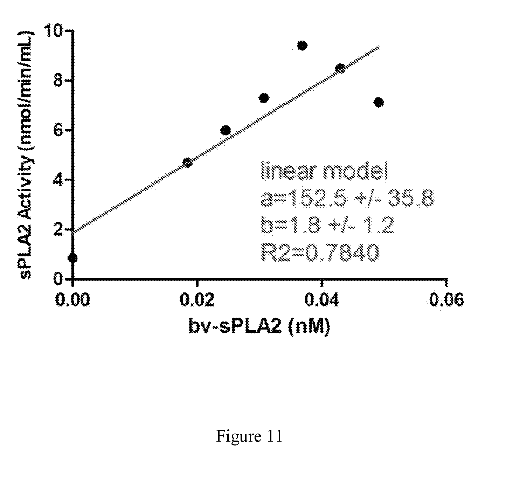

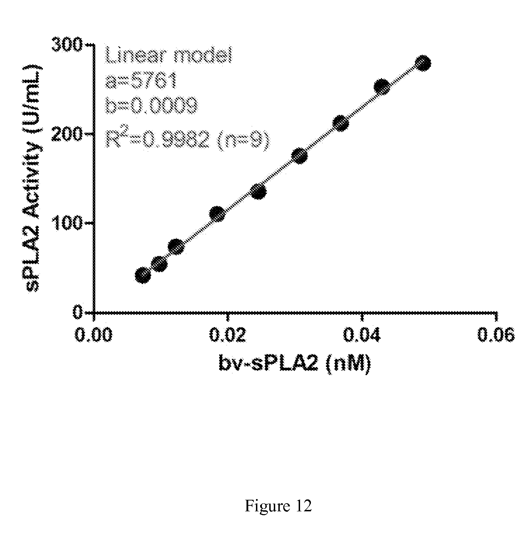

sPLA2 activity was determined using either the method of the invention or published methods in 30 µL of human serum (same sample for all the experiment) spiked with increasing amounts of bv-sPLA2 enzyme (same serial dilutions for all the experiment). Standard curves were then plotted for both experiments. As illustrated in figure 11 and 12, linearity of published method was restricted to 0.03 nM of bv-sPLA2, whereas linearity was observed within the whole tested sPLA2 range with the method of the invention.

### 5.3-Reproducibility and repeatability

Reproducibility (inter-assay variability) and repeatability (intra-assay variability) of published method were determined using a protocol similar to those used in example 4. Inter-assay CV for individual human plasma samples ranged from 28.3% to 32.1% with an average inter-assay CV of 30.2%.
Intra-assay CV for individual human plasma samples ranged from 4.6% to 40.9% with an average intra-assay CV of 25.0%.
As presented in example 4.3, average inter-assay and intra-assay variability are 5.24% and 8.46%, respectively.
The method of the invention is therefore much more precise and repeatable than previous published methods, with a 6-fold and 4-fold gain, respectively.

The lower number of pipetting steps, automatic initiation of the reaction, automatic analysis of the progress curve in the method of the invention explain the better reproducibility and repeatability, compared to previous published methods.

### 6- Determination of sPLA2 activity in samples from apparently healthy subject according to the method of the invention

We examined the sPLA2 activity distribution by age, gender and ethnicity in a total of 390 samples from apparently healthy males (n=240) and apparently healthy females (n=150). in the clinically relevant age range of 40-59 years.
The reference population was represented by the following ethnic backgrounds: black n=215, Caucasian n=108, Hispanic n=65, other = 2.

sPLA2 activity was measured according to the method of the invention.
30 µl of serum sample were added to 1 -hexadecanoyl-2-(1 -pyrenedecanoyl)-sn-glycero-3-phosphoglycerol stabilized on latex beads (1% beads suspension) in the presence of 200 µl of buffer (10 mM Tris-HCl pH 8.5. 0.5% BSA. 6mM EGTA and 100 mM NaCl) at 30°C in a 96-wells plate.
Background fluorescence emission was recorded for 90 sec in Fluostar Optima fluorimeter equipped with a stirring device and thermostat ruled at 30°C. The reaction was initiated by the injection of 20 µl of a CaCl2 solution (55 mM), automatically performed by the fluorimeter. Fluorescence emission was recorded for 90 sec and the progress curve was fitted using nonlinear regression. After subtraction of background level, final point at 1 min was determined from the obtained fitted curve equation. A standard calibration curve was obtained by plotting the final point at 1 min obtained for each of six sPLA2 Calibrators on the y-axis versus the sPLA2 concentration on the x-axis and final points at 1 min of each sample were then normalized using the standard calibration curve.
sPLA2 activity are expressed as U/ml. by converting the normalized fluorescence values into quantity (in nmol) of product liberated in one min. All samples were tested in duplicate.

In the total analyzed population, sPLA2 activity ranged from 10.6 to 79.9 U/mL, the geometric mean sPLA2 activity was 35.1U/mL and the median was 36.9 U/mL. The 95^{th} and 99^{th} percentiles were 46.1 and 52.5 for male and 61.6 and 72.4 for females, respectively.

The percentile distribution of sPLA2 activity by age, sex and race or ethnicity is presented in table 8. The age-adjusted mean sPLA2 activity in the population in the analyzed population is presented in table 9.
The reference interval calculated from the samples (central 90%) was found to be 29.6-54.0 U/mL for females and 19.6-51.2 U/mL for males.

Recent published studies (see references 5, 6, 7 and 9) have demonstrated an increased risk of cardiovascular disease (CVD) associated with sPLA2 activity values in the second and third tertiles (upper 67% of the studied populations) vs. the first tertile (lower 33% of the studied populations). Therefore, a more conservative approach for identifying individuals with a significant increased risk for CVD attributable to sPLA2 may be the 50th percentile value of the population.

In the current sPLA2 Activity test, this threshold value corresponds to 39.7 U/mL and 33.5 U/mL for female and male, respectively.

**Table 8: Distribution of sPLA2 activity among apparent/y healthy Male and Female. 40-59 years of age**

| **Percentile** | n | AM | GM | **0** | **1** | **10** | **20** | **30** | **40** | **50** | **60** | **70** | **80** | **90** | **95** | **100** |
|---|---|---|---|---|---|---|---|---|---|---|---|---|---|---|---|---|
| **Total** | 390 | 37.0 | 35.1 | 10.6 | 12.1 | 22.8 | 28.0 | 31.0 | 33.3 | 36.9 | 40.0 | 42.7 | 46.0 | 51.5 | 55.2 | 79.9 |
| | | | | | | | | | | | | | | | | |
| **Sex** | | | | | | | | | | | | | | | | |
| Female | 150 | 40.2 | 39.0 | 14.1 | 16.9 | 29.6 | 32.4 | 35.6 | 37.8 | 39.7 | 41.8 | 44.2 | 46.9 | 54.0 | 57.5 | 79.9 |
| Male | 240 | 35.0 | 31.0 | 10.6 | 11.5 | 19.6 | 26.5 | 29.1 | 31.1 | 33.5 | 37.6 | 41.9 | 45.5 | 51.2 | 54.2 | 63.3 |
| | | | | | | | | | | | | | | | | |
| **Age. years** | | | | | | | | | | | | | | | | |
| 40-44 | 105 | 40.1 | 39.3 | 22.3 | 24.0 | 30.3 | 33.1 | 36.2 | 37.8 | 39.4 | 41.1 | 43.9 | 45.0 | 50.3 | 55.4 | 62.9 |
| 45-49 | 120 | 35.4 | 33.3 | 12.2 | 13.1 | 19.5 | 26.7 | 29.1 | 31.3 | 32.9 | 36.4 | 41.3 | 46.2 | 52.8 | 56.5 | 79.9 |
| 50-54 | 118 | 37.9 | 36.1 | 10.6 | 11.9 | 23.9 | 28.7 | 29.8 | 34.3 | 39.2 | 42.0 | 44.9 | 47.1 | 51.6 | 53.7 | 63.3 |
| 55-59 | 47 | 32.2 | 29.7 | 11.1 | 11.5 | 15.1 | 20.0 | 26.7 | 30.5 | 32.0 | 34.1 | 37.9 | 41.5 | 46.8 | 54.1 | 62.1 |
| | | | | | | | | | | | | | | | | |
| **Ethnicity** | | | | | | | | | | | | | | | | |
| Black | 215 | 37.4 | 35.7 | 12.2 | 13.8 | 25.0 | 29.4 | 31.6 | 34.0 | 37.2 | 39.5 | 42.2 | 45.6 | 50.6 | 56.0 | 79.9 |
| Caucasian | 108 | 37.4 | 35.6 | 10.6 | 11.6 | 22.7 | 28.0 | 29.6 | 33.6 | 38.9 | 41.9 | 43.8 | 46.8 | 51.4 | 53.6 | 63.3 |
| Hispanic | 65 | 35.5 | 33.0 | 11.1 | 11.6 | 15.6 | 27.5 | 30.4 | 32.2 | 34.8 | 39.1 | 42.3 | 45.1 | 53.1 | 54.4 | 62.1 |
| Other | 2 | 26.1 | 26.1 | 26.0 | 26.0 | 26.0 | 26.0 | 26.0 | 26.1 | 26.1 | 26.1 | 26.1 | 26.1 | 26.1 | 26.1 | 26.1 |

**Table 9: Age-adjusted mean sPLA2 activity among apparently healthy Male and Female. 40-59 years of age**

| | **Black** | | **Caucasian** | | **Hispanic** | |
|---|---|---|---|---|---|---|
| | Mean | SEM | Mean | SEM | Mean | SEM |
| **Male** | 37.0 | 0.9 | 38.8 | 2.3 | 39.4 | 1.6 |
| All | 33.3 | 0.7 | 32.7 | 1.2 | 29.9 | 1.8 |
| 40-44 | 38.1 | 0.9 | 39.7 | 2.2 | 39.4 | 2.1 |
| 45-49 | 27.4 | 1.1 | 27.2 | 1.6 | 22.6 | 2.5 |
| 50-54 | 29.2 | 2.6 | 29.4 | 2.3 | 24.7 | 2.6 |
| 55-59 | | | | | | |
| | | | | | | |
| **Female** | | | | | | |
| All | 44.6 | 1.3 | 43.9 | 1.4 | 42.9 | 2.0 |
| 40-44 | 41.8 | 1.8 | 43.5 | 2.7 | 44.3 | 4.0 |
| 45-49 | 47.0 | 2.1 | 46.4 | 1.8 | 49.4 | 2.5 |
| 50-54 | 47.2 | 3.6 | 41.0 | 2.6 | 36.0 | 1.8 |
| 55-59 | 42.1 | 4.2 | 51.6 | 0.0 | 28.1 | 0.4 |

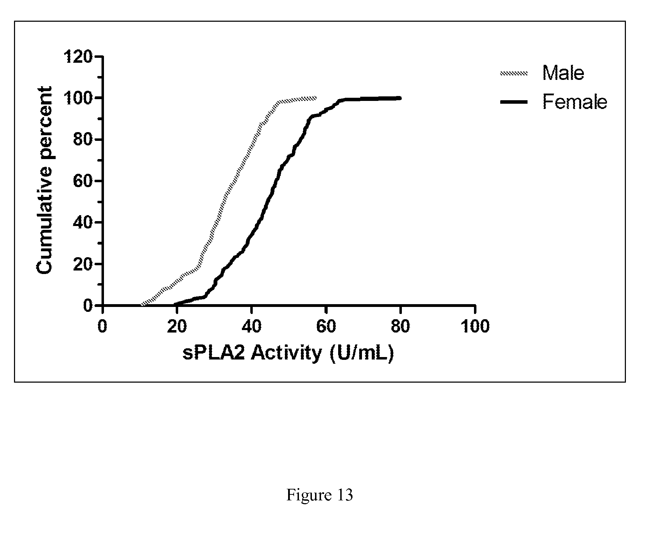

The cumulative percent distribution in male and female is presented in figure 13.

### REFERENCES

**1-** Berg OG, Gelb MH, Tsai MD, Jain MK. Interfacial enzymology: the secreted phospholipase A(2)-paradigm. Chem Rev, 2001 Sep;101(9):2613-54.
**2-** Kugiyama K. et al. 1999. Circulating levels of secretory type II phospholipase A(2) predict coronary events in patients with coronary artery disease. Circulation, 1999 Sep 21;100(12):1280-4.
**3-** Kugiyama K, Ota Y, Sugiyama S, Kawano H, Doi H, Soejima H, Miyamoto S, Ogawa H, Takazoe K, Yasue H. Prognostic value of plasma levels of secretory type II phospholipase A2 in patients with unstable angina pectoris. Am J Cardiol, 2000 Oct 1;86 (7):718-22.
**4-** Liu PY, Li YH, Tsai WC, Chao TH, Tsai LM, Wu HL, Chen JH. Prognostic value and the changes of plasma levels of secretory type II phospholipase A2 in patients with coronary artery disease undergoing percutaneous coronary intervention. Eur Heart J., 2003 Oct;24 (20):1824-32.
**5-** Mallat Z, Steg PG, Benessiano J, Tanguy ML, Fox KA, Collet JP, Dabbous OH, Henry P, Carruthers KF, Dauphin A, Arguelles CS, Masliah J, Hugel B, Montalescot G, Freyssinet JM, Asselain B, Tedgui A. Circulating secretory phospholipase A2 activity predicts recurrent events in patients with severe acute coronary syndromes. J Am Coll Cardiol., 2005 Oct 4;46(7):1249-57.
**6-** W. Koenig, C.Y. Vossen, Z. Mallat, H. Brenner, A. Tedgui, D. Rothenbacher. Association Between Type II Secretory Phospholipase A2 Plasma Concentrations and Activity and Cardiovascular Events in Patients with Coronary Heart Disease. European Society of Cardiology Meeting, Munich. Germany. 2008. P3664
**7-** Tabassome Simon, Joelle: Benessiano, Murielle Mary Krause, Eva Ninio, Gabriel Steg, Gueret Pascal, Elodie Drouet, Alain Tedgui, Nicolas Danchin, Ziad Mallat on behalf of Fast-MI study. Impact of circulating secretory PhosphoLipase A2 (sPLA2) and Lipoprotein-associated phospholipase A2 (Lp-PLA2) activities on 6 months-survival, recurrent AMI and incident stroke in patients with AMI. European Society of Cardiology Meeting, Munich. Germany. 2008. P1317
**8-** Boekholdt SM. Keller TT. Wareham NJ. Luben R. Bingham SA. Day NE. Sandhu MS. Jukema JW. Kastelein JJ. Hack CE. Khaw KT. Serum levels of type II secretory phospholipase A2 and the risk of future coronary artery disease in apparently healthy men and women: the EPIC-Norfolk Prospective Population Study. Arterioscler Thromb Vase Biol. 2005 Apr;25(4):839-46.
**9-** Mallat Z, Benessiano J, Simon T, Ederhy S, Sebella-Arguelles C, Cohen A, Huart V, Wareham NJ, Luben R, Khaw KT, Tedgui A, Boekholdt SM. Circulating secretory phospholipase A2 activity and risk of incident coronary events in healthy men and women: the EPIC-Norfolk study. Arterioscler Thromb Vase Biol., 2007 May;27(5): 177-83. Epub 2007 Feb 15.
**10-** Tsimikas S; Mallat Z.; Philippa J Talmud; John J Kastelein; Nicholas J Wareham; Elizabeth R Miller; Joseph L Witztum; Kay-Tee Khaw; Boekholdt S. M. Elevated Oxidized Phospholipids on Apolipoprotein B-100 Particles Predict 6-Year Cardiovascular Events in the Epic-Norfolk Study: Potentiation of Risk with Lipoprotein-Associated (Lp-PLA2) and Soluble Phospholipase A2 (sPLA2) Activity. American Heart Association
**11-** Lambeau G, Gelb MH. Biochemistry and physiology of mammalian secreted phospholipases A2. Annu Rev Biochem. 2008;77:495-520. Review
**12-** Kim Y., Lichtenbergova L., Snitko Y, and Cho W. A phospholipase A2 kinetic and binding assay using phiospholipid-coated hydrophobic beads. Analytical biochemistry 1997, 205: 109-116.
**13-** Thuren T., Virtanen JA, Lalla M, Kinnunen PK., Fluorometric assay for phospholipase A2 in serum. 1985 Clin. Chem. 31:714-7

## Claims

1. A method for measuring a calcium-dependent phospholipase A1 or A2 enzymatic activity in a sample, comprising the following steps:
a) contacting said sample with a solid phase coated with a fluorochrome-labelled phospholipase A1 or A2 substrate, wherein the molecular coverage is in the range 8 to 11 molecules / nm2 in conditions where calcium-dependent phospholipase A1 or A2 enzymatic activity is blocked.
b) reading the fluorescence emission overtime
c) adding a solution to initiate calcium-dependent phospholipase A1 or A2 enzymatic activity
d) reading the fluorescence emission overtime.

2. The method according to claim **1,** wherein the sample is selected from urine, serum, plasma sample, whole blood, bronchoalveolar lavage fluid, sputum, synovial fluid, amniotic fluid, peritoneal fluid, cerebrospinal fluid, pleural fluid, pericardial fluid and alveolar macrophages.

3. The method according to claim **1** or **2,** wherein step a) is performed in the presence of at least one divalent ions chelating agent.

4. The method according to claim **3,** wherein the concentration of the divalent ions chelating agent is from 1 mM to 20 mM.

5. The method according to anyone of claim **1** to **4,** wherein the solution that initiates calcium-dependent phospholipase A1 or A2 enzymatic activity comprises an excess of Ca2+ divalent ions.

6. The method according to claim **5,** wherein the concentration of Ca2+ divalent ions in the solution is from 1 to 10 mM.

7. The method according to anyone of claim **1** to **6,** wherein said substrate comprises a hydrophobic moiety, a phosphate moiety and a fluorescent moiety attached to the hydrophobic moiety either directly or via an optional linker.

8. The method according to anyone of claim **1** to **7,** wherein said substrate is a glycerophospholipid.

9. The method according to anyone of claim **1** to **8,** wherein said substrate is labelled on the terminal end of the sn-1 fatty acyl chain and/or on the terminal end of the sn-2 fatty acyl chain.

10. The method according to anyone of claim **1** to **8,** wherein the labelled substrate is labelled on the sn-1 fatty acid chain with a donor fluorophore and on the sn-2 fatty acid chain with an acceptor fluorophore or inversely.

11. The method according to anyone of claim **1** to **8,** wherein the labelled substrate is labelled on the sn-1 fatty acid chain with a fluorophore and on the sn-2 fatty acid chain with an quencher or inversely.

12. A kit comprising:
- a first container comprising a solid phase coated with a fluorochrome-labelled phospholipase A1 or A2 substrate, wherein the molecular coverage is in the range 8 to 11 molecules / nm2,
- a second container comprising an assay buffer comprising at least a buffer solution, one or more divalent ions chelating agent, and bovine serum albumin.
- a third container comprising a solution that initiates calcium-dependent phospholipase A1 or A2 enzymatic activity, said solution comprising Ca2+ divalent ions.

13. The kit according to claim **12** further comprising controls and/or calibrators.

14. A method of identifying a subject having or at risk of having or developing a sPLA2 activity-linked disease, comprising measuring the sPLA2 enzymatic activity in a sample from the subject according to the method of anyone of claims **1** to **11.**

15. The method according to claim **14,** wherein said sPLA2 activity-linked disease is a cardiovascular disease and/or a cardiovascular event.
